(19) Europäisches Patentamt
European Patent Office
Office européen des brevets

(11) Publication number : **0 082 546 B2**

# (12) NEW EUROPEAN PATENT SPECIFICATION

(45) Date of publication of the new patent specification : **25.03.92 Bulletin 92/13**

(51) Int. Cl.$^5$ : **A61K 31/415,** // (A61K31/415, 31:35)

(21) Application number : **82201545.9**

(22) Date of filing : **06.12.82**

(54) **Pharmaceutical mixture and method for making the mixture.**

The file contains technical information submitted after the application was filed and not included in this specification

(30) Priority : **23.12.81 GB 8138755**

(43) Date of publication of application : **29.06.83 Bulletin 83/26**

(45) Publication of the grant of the patent : **14.08.85 Bulletin 85/33**

(45) Mention of the opposition decision : **25.03.92 Bulletin 92/13**

(84) Designated Contracting States : **DE GB NL SE**

(56) References cited :
**DE-A- 1 792 799**
**DE-A- 2 223 237**
**DE-A- 2 425 281**
**GB-A- 1 087 842**
**US-A- 3 975 536**
**US-A- 4 053 628**
**"Dictionnaire vidal", 1979, O.V.P., pages 1199-1200, Paris (FR)**
**UNLISTED DRUGS, vol. 18, no. 4, April 1966, page 33e, Chatham, New Jersey (USA)**
**G.ERHART et al., Arzneimittel Band 2, 1972; p. 150**
**ROTE LISTE, 1980; nos. 71039, 71034b, 67122, 71071, 71085, 67237, 21024**
**Kurzes Lehrbuch der Pharmakologie, Kuschinsky & Luellmann, Stuttgart (DE), 1974; p. 41**
**Dr. B.HELWIG, Moderne Arzneimittel, 4th ed., 1972; p. 1220(3)**
**Ullmanns Enzyklopädie der technischen Chemie, 4th ed., vol. 12; p. 652**
**Clinical Allergy, 1973, vol. 3; pp. 283-288**
**ROTE LISTE, 1967; "Titelseite" and p. 872**
**ROTE LISTE, 1977/78; nos. 71037, 71039**
**VIP Lomupren, 4/81**
**Cardiovascular Clinics, vol. 2, no. 3, 1971; pp. 37-54**
**Acta Allergologica, 1972, no. 27; pp. 307-320**
**The Mast Cell, Pitman Medical Co.Ltd., 1979; pp. 816-819**

(56) References cited :
**Martindale's Extra Pharmacopoeia, 27th ed., The Pharmaceutical Press, 1977; pp. 2, 27, 36**
**Textbook of Pharmacology, 2nd ed., Blackwell Scientific Publications; pp. 11.28-11.32**

(73) Proprietor : **FISONS plc**
**Fison House Princes Street**
**Ipswich Suffolk IP1 1QH (GB)**

(72) Inventor : **Simpson, Wilfrid Tord**
**"Roa" Cottesmore Road**
**Birley Oakham Leicestershire (GB)**
Inventor : **Stevenson, Neil Arthur**
**16 Longcliffe Gardens Nanpantan**
**Loughborough Leicestershire (GB)**

(74) Representative : **Craig, Christopher Bradberry et al**
**Fisons plc 12 Derby Road**
**Loughborough Leicestershire LE11 0BB (GB)**



Jouve, 18, rue Saint-Denis, 75001 PARIS

## Description

This invention relates to novel mixtures.

Sodium cromoglycate has been known for many years to be useful in the treatment of allergic conditions, notably of the lung, but also of the nose (see Dictionnaire Vidal, 1979, O-V-P., Paris, pages 1199-1200 'LOMUSOL'). It has also, separately, been known to use vasoconstrictors, e.g. xylometazoline hydrochloride aqueous solution (see Unlisted Drugs, Vol- 18 No- 4, April 1966, Chatham, New Jersey, page 33e) or oxymetazoline hydrochloride aqueous solution, to relieve nasal congestion. However the use of vasoconstrictors at therapeutically effective doses has, in certain cases, been found to cause local stinging or burning, sneezing and dryness of the nasopharynx. Use of relatively high doses of vasoconstrictors may also cause rebound congestion and drug induced rhinitis.

We have now found that the concentration of xylometazoline can be radically reduced, while still retaining its therapeutic duration and effect, when used in admixture with sodium cromoglycate. The reduction in concentration, and hence dosage, of the xylometazoline removes or mitigates the risk of unwanted side effects. Surprisingly also the mixture is more efficacious than sodium cromoglycate alone and the presence of the xylometazoline tends to prolong the action of the sodium cromoglycate. We have also found that the cationic xylometazoline remains in solution with the anionic cromoglycate. This is particularly surprising as many salts of the cromoglycate anion, e.g. the calcium and magnesium salts, are extremely insoluble in water.

According to the invention we provide an aqueous solution containing from 0.5 to 58 w/v of sodium cromoglycate and from 0.01 to 0.05% w/v of xylometazoline, or a pharmaceutically acceptable salt thereof, measured as the hydrochloride.

We prefer the solution to be clear.

The solution contains preferably about 1 or 2% w/v of the sodium cromoglycate.

The solution contains desirably about 0.025% w/v of xylometazoline, measured as the hydrochloride.

The composition may also contain an effective proportion of a pharmaceutically acceptable chelating or sequestering agent. Suitable sequestering or chelating agents include citric, tartaric or phosphoric acid, and amino carboxylate compounds, e.g. ethylenediamine tetraacetic acid or its salts, e.g. its calcium salt, its calcium-sodium salt, or more preferably its di-sodium salt.

The composition may also contain an effective proportion of a pharmaceutically acceptable chelating or sequestering agent. Suitable sequestering or chelating agents include citric, tartaric or phosphoric acid, and amino carboxylate compounds, preferably ethylenediamine tetraacetic acid or its salts, e.g. its calcium salt, its calciumsodium salt, or more preferably its di-sodium salt.

The concentration of the chelating or sequestering agent may vary considerably, but in any case should be such as to ensure that no precipitate of metal salts of the cromoglycate anion occurs. A suitable concentration of chelating or sequestering agent may be from 0.05 to 0.1 % w/v. When a very low concentration, i.e. less than 0.40, preferably less than 0.32 p.p.m., of 'metal ions' are present, for example when the solution contains less than 0.08 p.p.m. of ionic iron and less than 0.25 p.p.m. of ionic zinc, the chelating or sequestering agent may if desired be dispensed with. When a chelating or sequestering agent is used the concentration of 'metal ions' is preferably less than 20 p.p.m. and more preferably less than 10 p.p.m.

By the term 'metal ions' we mean ions of metals in groups IIa, Ib, IIb, and IVb of the periodic table and of the transition metals. Specific 'metal ions' which are detrimental, in excessive concentrations, i.e. above 20 p.p.m., to the compositions of the invention are Pb++, Ca++, Mg++ and in particular Fe++, Fe+++ and Zn++ ions.

The composition may if desired contain an effective proportion, e.g. from 0.001 to 0.10% w/v, of a pharmaceutically acceptable preservative or sterilising agent. Suitable preservatives include pharmaceutically acceptable quaternary ammonium compounds, for example, the alkyl benzyl di-methyl ammonium chlorides and mixtures thereof, e.g. that known generically as 'Benzalkonium chloride'. This latter consists of a mixture of compounds of formula

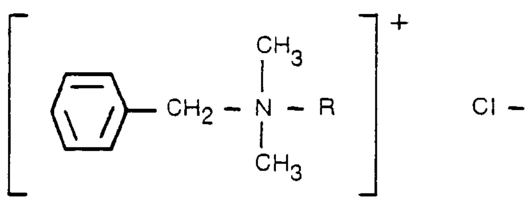

in which R is an alkyl group $C_8H_{17}$ to $C_{18}H_{37}$. We particularly prefer to use a mixture of such compounds in which R is $C_{10}H_{21}$ to $C_{14}H_{29}$ and especially those specific compounds in which R is $C_{12}H_{25}$ and $C_{14}H_{29}$. "Benzalkonium chloride', and the compounds of the formula given above, may be used at a concentration of from 0.005 to 0.10 preferably 0.005 to 0.05, e.g. about 0.01% w/v.

The composition may also contain conventional excipients, e.g. sodium chloride, and buffers, e.g. sodium dihydrogen orthophosphate (sodium acid phosphate B.P.) and di-sodium hydrogen phosphate (sodium phosphate B.P.). The proportion and concentration of excipients and buffers may be varied within fairly wide ranges, provided the resulting solution is stable and non-irritant when applied to the appropriate tissues. For maximum stability the preferred pH is from 4.5 to 7.5, more preferably 4.5 to 6.5 and especially 5 to 5.5, with minimal buffering to avoid tissue irritation. The maximum total concentration of excipients and buffers is preferably less than 5% w/v and more preferably less than 2% w/v. We prefer to use a buffered isotonic solution.

The composition, when it does not contain a preservative, may be made up using conventional techniques, by dissolving the components in water, filtering the solutions and sterilising the filtrate. More specifically the compositions may be made by dissolving the chelating or sequester ing agent (if included) and the xylometazoline salt, e.g. the hydrochloride, in freshly distilled water, adding the excipients, buffers etc. to the aqueous solution of chelating or sequestering agent etc., adding the sodium cromoglycate to the resulting solution, stirring, filtering and then sterilising the composition by autoclaving, for example at a temperature of about 115°C for about 30 minutes. The autoclaving of the product may, if desired, be omitted when the composition is produced by sterile filtration into a previously sterilised container under aseptic conditions.

Compositions which contain preservatives may be made up by mixing two aqueous solutions of equal volumes one containing twice the desired final concentration of the sodium cromoglycate, chelating or sequestering agent, and other additives, and the other containing twice the desired final concentration of the preservative and the xylometazoline salt. The resulting mixture may then be filtered under sterile conditions. Surprisingly we find that mixture of a solution of the anionic cromoglycate with a solution of a cationic quaternary ammonium preservative and the xylometazoline does not precipitate all the preservative or the xylometazoline from the mixed solution.

The solutions according to the invention should be protected from light during manufacture and storage. The solutions may be filled into opaque bottles containing from about 10 to 30 mls of solution and adapted to be fitted with a spray pump designed to deliver the desired dosage.

The compositions may be used in the treatment of hay fever by administration to the nose, e.g. as a nasal spray. For nasal application we prefer a composition containing about 2% w/v of sodium cromoglycate. The dosage given will vary with the particular compositions used and the condition to be treated. In general however a dosage of about 0.13 to 0.16 ml (i.e. about 3-2 mg of sodium cromoglycate) for each nostril from 2 to 6 times a day is indicated. The compositions may also be used in the treatment of conditions of the eye, e.g. allergic conditions of the conjunctiva, by administration of 1 or 2 drops to the affected eye up to 10 times, e.g. about 6 times per day.

The invention is illustrated but in no way limited by the following Examples:-

Example 1

| Preserved Nasal Spray Solution | |
|---|---|
| Sodium cromoglycate B.P. | 2.0% w/v |
| Di-sodium edetate B.P. | 0.01% w/v |
| Benzalkonium chloride solution USP 50% w/v | 0.029% v/v |
| Xylometazoline hydrochloride | 0.025% w/v |
| Sterile distilled water | to 100% |

In 50 parts by volume of water dissolve first the disodium edetate and then the sodium cromoglycate. In 30 parts by volume of water dissolve in the benzalkonium chloride solution and the xylometazoline hydrochloride. Mix the solutions and make up to 100 parts by volume with water. The mixture is then stirred, filtered and filled into opaque polyethylene bottles fitted with a screw cap. The water used in particularly pure in that it contains very low concentrations of 'metal ions'

Example 2

Fifteen patients suffering from allergic rhinitis were treated in a double-blind group comparison with either the composition of Example 1, or the composition of Example 1 from which the xylometazoline hydrochloride had been omitted. The patients were asked to keep a diary card score of the degree of nasal symptoms experienced during the first three days of the treatment. The diary card was scored 0 = no symptoms, 1 = mild, 2 = moderate, 3 = severe and 4 = very severe symptoms. The dosage was a spray of 0.13 mls of solution into each nostril 4 times a day. The patients entered the trial during a period when there was a high pollen count. The mean score for the treatment with the composition containing xylometazoline hydrochloride was 0.47 (7 patients) and for the composition containing no xylometazoline was 2. 10 (8 patients), i.e. the mixture according to the invention was about 4 times more effective than the other formulation in this test.

## Claims

1. An aqueous solution containing from 0.5 to 5% w/v of sodium cromoglycate and from 0.01 to 0.05% w/v of xylometazoline, or a pharmaceutically acceptable salt thereof, measured as the hydrochloride.

2. An aqueous solution according to Claim 1 containing 0.025% w/v of xylometazoline measured as the hydrochloride.

3. An aqueous solution according to Claim 1 or 2 comprising an effective amount of a pharmaceutically acceptable chelating or sequestering agent and an effective amount of a pharmaceutically acceptable preservative.

4. A mixture according to Claim 3 comprising from 0.005 to 0.05% w/v of benzalkonium chloride and from 0.005 to 0.1% w/v of the disodium salt of ethylenediamine tetracetic acid.

5. A mixture according to any one of the preceding claims having a pH of from 4.5 to 6.5.

6. A mixture according to Claim 1 comprising an aqueous solution of 2.0% w/v sodium cromoglycate, 0.01% w/v disodium edetate, 0.015% w/v benzalkonium chloride and 0.025% w/v of xylometazoline hydrochloride.

7. A method of making a mixture according to Claim 3, which comprises mixing a first solution containing the sodium cromoglycate and the chelating or sequestering agent with a second solution containing the xylometazoline salt and the preservative and filtering the resulting mixture.

## Revendications

1. Solution aqueuse contenant 0,5 à 5% p/v de cromoglycate sodique et 0,01% à 0,05% p/v de xylométazoline, ou d'un sel pharmaceutiquement acceptable de celle-ci, à mesurer en chlorhydrate.

2. Solution aqueuse suivant la revendication 1, contenant 0,025% p/v de xylométazoline, à mesurer en chlorhydrate.

3. Solution aqueuse suivant la revendication 1 ou 2, comprenant une quantité efficace d'un agent chélatant ou séquestrant pharmaceutiquement acceptable et une quantité efficace d'un conservateur pharmaceutiquement acceptable.

4. Mélange suivant la revendication 3, comprenant 0,005 à 0,05% p/v de chlorure de benzalkonium et 0,005 à 0,1% p/v du sel disodique de l'acide éthylènediamine tétraacétique.

5. Mélange suivant l'une quelconque des revendications précédentes, ayant un pH de 4,5 à 6,5.

6. Mélange suivant la revendication 1, comprenant une solution aqueuse de 2,0% p/v de cromoglycate sodique, de 0,01% p/v d'édétate disodique, de 0,015% p/v de chlorure de benzalkonium et de 0,025% p/v de chlorhydrate de xylométazoline.

7. Procédé de préparation d'un mélange suivant la revendication 3, qui comprend l'action de mélanger une première solution contenant le cromoglycate sodique et l'agent chélatant ou séquestrant avec une deuxième solution contenant le sel de xylométazoline et le conservateur et la filtration du mélange résultant.

## Patentansprüche

1. Wässerige Lösung enthaltend 0,5 bis 5 % G/V Natriumcromoglykat und 0,01 bis 0,05 % G/V Xylometazolin oder ein pharmazeutisch annehmbares Salz hievon, gemessen als das Hydrochlorid.

2. Wässerige Lösung nach Anspruch 1 enthaltend 0,025 % G/V Xylometazolin, gemessen als das Hydrochlorid.

3. Wässerige Lösung nach Anspruch 1 oder 2 umfassend eine wirksame Menge eines pharmazeutisch annehmbaren Chelatbildners oder Sequestriermittels und eine wirksame Menge eines pharmazeutisch annehmbaren Konservierungsmittels.

4. Mischung nach Anspruch 3 umfassend 0,005 bis 0,05 % G/V Benzalkoniumchlorid und 0,005 bis 0,1 % G/V des Dinatriumsalzes von Äthylendiamintetraessigsäure.

5. Mischung nach einem der vorhergehenden Ansprüche mit einem pH von 4,5 bis 6,5.

6. Mischung nach Anspruch 1 umfassend eine wässerige Lösung von 2,0 % G/V Natriumcromoglykat, 0,01 % G/V Dinatriumedetat, 0,015 % G/V Benzalkoniumchlorid und 0,025 % G/V Xylometazolinhydrochlorid.

7. Verfahren zum Herstellen einer Mischung nach Anspruch 3, das das Mischen einer das Natriumcromoglykat und den Chelatbildner oder das Sequestriermittel enthaltenden ersten Lösung mit einer das Xylometazolinsalz und das Konservierungsmittel enthaltenden zweiten Lösung und das Filtrieren der erhaltenen Mischung umfaßt.